# EUROPEAN PATENT APPLICATION

(11) **EP 1 806 594 A2**
(43) Date of publication of application: **11.07.2007**
(21) Application number: 07000170.6
(22) Date of filing: 05.01.2007
(51) Int. Cl.: G01S 7/52, A61B 8/08

(54) **Ultrasound system and method of displaying ultrasound image**

(30) Priority: 06.01.2006 KR 20060001591
(71) Applicant: MEDISON CO., LTD., Kangwon-do 250-870 (KR)
(72) Inventor: Kim, Hyoung Jin, Discusser & Medison Building, Seoul 135-280 (KR); Hyun, Dong Gyu, Discusser & Medison Building, Seoul 135-280 (KR); Ahn, Chi Young, Discusser & Medison Building, Seoul 135-280 (KR)
(74) Representative: Schmid, Wolfgang

(57) **Abstract**

An ultrasound system, which includes an ultrasound diagnostic unit for transmitting ultrasound signals to a target object in a periodic motion and forming ultrasound images based on receive signals reflected from the target object comprises a first storage unit for storing the ultrasound images provided from the ultrasound diagnostic unit together with the reception times of the receive signals; a cycle setting unit for setting a cycle of motion of the target object; a processor for selecting the ultrasound images within a specific cycle among the ultrasound images stored in the first storage unit based on the reception times of the receive signals corresponding to the ultrasound images and start and end times of the specific cycle provided from the cycle setting unit, the processor further being configured to form an equal number of ultrasound images within each cycle based on the number of the selected ultrasound images; and a display unit for simultaneously displaying the ultrasound images within each cycle.

## Description

The present application claims priority from Korean Patent Application No. 10-2006-0001591 filed on January 6, 2006, the entire subject matter of which is incorporated herein by reference.

### BACKGROUND

### 1. Field

The present invention generally relates to ultrasound systems, and more particularly to an ultrasound system and a method of displaying ultrasound images relating to the periodic motion of an object.

### 2. Background

An ultrasound diagnostic system has become an important and popular diagnostic tool due to its wide range of applications. Specifically, due to its non-invasive and non-destructive nature, the ultrasound diagnostic system has been extensively used in the medical profession. Modern high-performance ultrasound diagnostic systems and techniques are commonly used to produce two or three-dimensional (2D or 3D) diagnostic images of a target object. The ultrasound diagnostic system generally uses a probe including an array transducer having a plurality of transducer elements to transmit and receive ultrasound signals. The ultrasound diagnostic system forms ultrasound images of the internal structures of the target object by electrically exciting the transducer elements to generate ultrasound pulses that travel into the target object. The ultrasound pulses produce ultrasound echoes since they are reflected from a discontinuous surface of acoustic impedance of the internal structure, which appears as discontinuities to the propagating ultrasound pulses. Various ultrasound echoes return to the array transducer and are converted into electrical signals, which are amplified and processed to produce ultrasound data for forming an image of the internal structure of the target object.

The heart acts as a pump to circulate blood throughout the entire body. Thus, the heart continually repeats to contract and relax. The pumping action of the heart is performed by the contraction of cardiac muscles. At every heartbeat, small electric currents are generated so as to flow into the body. Such generated currents change the electric potential distribution on the surface of the body. Electrocardiogram (ECG) is a recording of such electric currents generated during the heart activity, which are measured at an appropriate portion on the surface of the body and then amplified.

Different diagnoses may be conducted according to the doctors' instructions when using the electrocardiogram as a clinical test for heart disease. For example, a diagnosis may determine that a patient is normal even though he or she has a disease. Further, a diagnosis may determine that a patient is not normal even though he or she has no disease. Thus, there is a strong need for an accurate analysis method of analyzing the results of electrocardiogram measurements.

### BRIEF DESCRIPTION OF THE DRAWINGS

Arrangements and embodiments may be described in detail with reference to the following drawings in which like reference numerals refer to like elements and wherein:

FIG. 1 illustrates an ultrasound system constructed in accordance with one embodiment of the present invention;
FIG. 2 is a graph illustrating ECG waveforms;
FIG. 3 illustrates an example of ultrasound images within a cycle of the heartbeat stored in a cine memory;
FIG. 4 illustrates an example of ultrasound images within a cycle of the heartbeat displayed at the same time;
FIG. 5 illustrates an example of ECG waveforms as well as storing and displaying ultrasound images; and
FIG. 6 illustrates an example of displaying ultrasound images in the same order in the respective ECG cycles.

### DETAILED DESCRIPTION

A detailed description may be provided with reference to the accompanying drawings. One of ordinary skill in the art may realize that the following description is illustrative only and is not in any way limiting. Other embodiments of the present invention may readily suggest themselves to such skilled persons having the benefit of this disclosure.

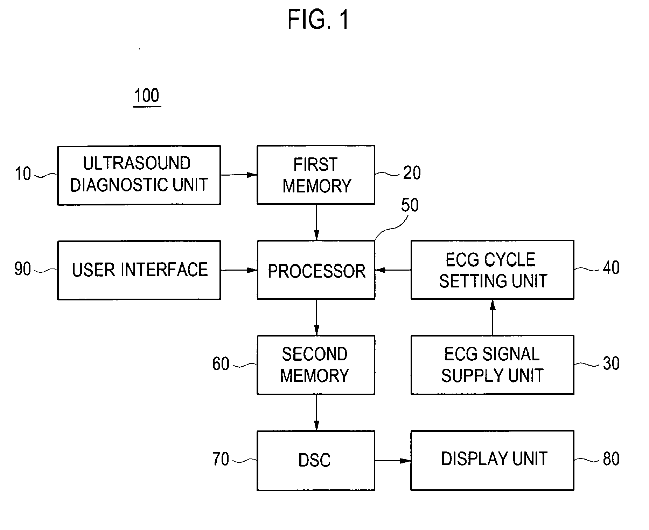

One embodiment of the present invention will be described below with reference to the accompanying drawings. FIG. 1 illustrates an ultrasound system, which is constructed in accordance with one embodiment of the present invention. However, it should be expressly noted herein that other arrangements may also be used.

As shown in FIG. 1, an ultrasound system 100 of the present invention may include an ultrasound diagnostic unit 10, a first memory 20, an ECG signal supply unit 30, an ECG cycle setting unit 40, a processor 50, a second memory 60, a digital scan converter (DSC) 70, a display unit 80 and a user interface 90. The first memory 20 and the second memory 60 can be configured with a single memory.

The ultrasound diagnostic unit 10 transmits ultrasound signals to an object in a periodic motion (e.g., heart) and provides ultrasound images at every moment of receiving signals reflected from the heart. The ultrasound image can be a 2 or 3-dimensional image. The ultrasound images provided by the ultrasound diagnostic unit 10 are stored in the first memory 20 together with the reception times of the receive signals.

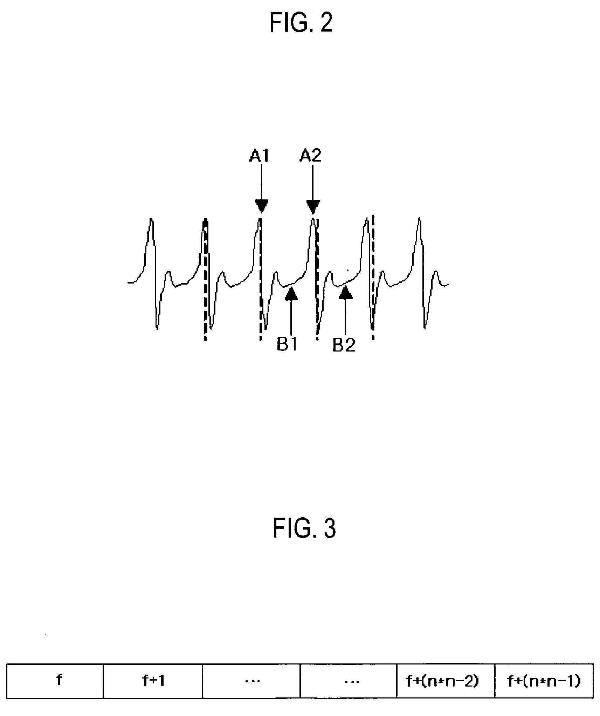

The ECG signal supply unit 30 receives electrocardiogram (ECG) signals from an ECG measuring apparatus and provides the ECG signals to the ECG cycle setting unit 40. The ECG signals can be digital signals, which are indicative of the magnitudes of electric potential. The ECG cycle setting unit 40 analyzes the ECG signals during a predetermined time period to set an ECG cycle, wherein said predetermined time period preferably ranges from 0.05 seconds to 3 seconds from a first reception time of ECG signals. The ECG cycle can be measured by using various methods. For example, in an ECG graph shown in FIG. 2, one ECG cycle can be set to a time interval between neighboring peaks A1 and A2 or between neighboring points B1 and B2 in the same phase. When the ECG cycle is set, the ECG cycle setting unit 40 transmits time information of start and end of each ECG cycle to the processor 50.

The processor 50 sets the ultrasound images stored in the first memory 20, which have the reception times approximately equal to the start and end of each ECG cycle, to be a start image and an end image of each ECG cycle.

In order to display n x n ultrasound images in each ECG cycle, the processor 50 counts the number of ultrasound images in each ECG cycle. If the number of ultrasound images within an ECG cycle is larger than n x n, then extra ultrasound images (except n x n ultrasound images) are removed. In such a case, differences between the reception times of the receive signals corresponding to the neighboring ultrasound images are calculated. Then, one of the neighboring ultrasound images having the smallest difference is first removed. Thereafter, extra ultrasound images are removed in such a manner that one of the neighboring ultrasound images having the smallest difference among the remaining ultrasound images is removed. If the number of ultrasound images in an ECG cycle is smaller than n x n, then supplementary ultrasound images are produced to make n x n ultrasound images within an ECG cycle. In such a case, differences between the reception times of the receive signals corresponding to the neighboring ultrasound images are calculated. Then, a new ultrasound image between the neighboring ultrasound images having the largest difference is interpolated and supplementary ultrasound images are produced in such a manner.

The second memory 60 stores n x n ultrasound images corresponding to each ECG cycle, which are provided from the processor 50. FIG. 3 shows an example of storing n x n ultrasound images within an ECG cycle in the second memory 60.

The DSC 70 scan-converts the ultrasound images, which are stored in the second memory 60, and then provides the scan-converted ultrasound images to the display unit 80.

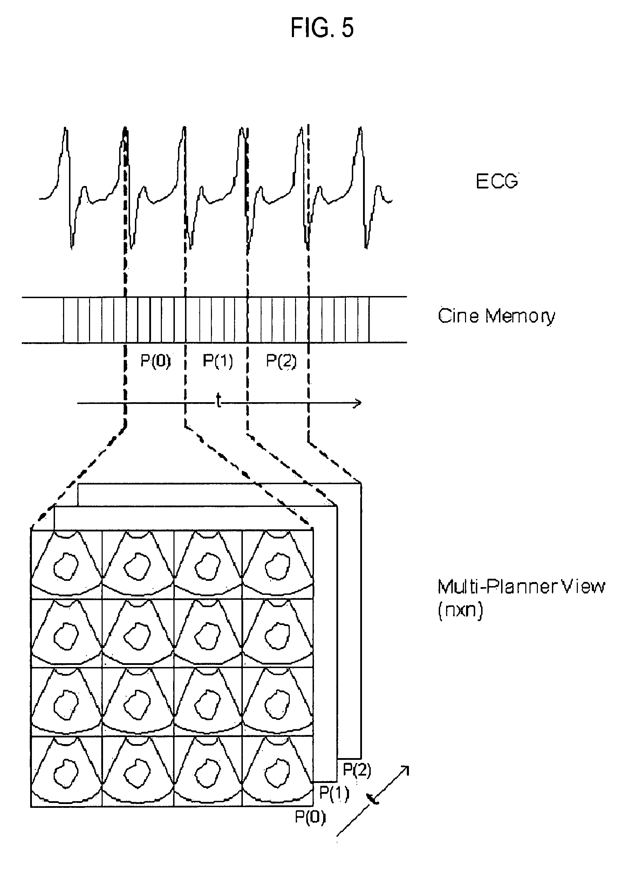

The display unit 80 displays the scan-converted ultrasound images provided from the DSC 70 such that n x n ultrasound images within one cycle of the heartbeat are displayed in the same time. The display unit 80 may display the ultrasound images within one cycle of the heartbeat on the screen of the display unit 80, which is divided into n x n areas, respectively. Also, the display unit 80 may display the respective ultrasound images on the screens of the n x n monitors. FIG. 5 shows an example of ECG waveforms as well as storing and displaying the ultrasound images. In FIG. 5, the ultrasound images in the respective ECG cycles P(0), P(1), P(2) ... are stored and displayed at specified time intervals.

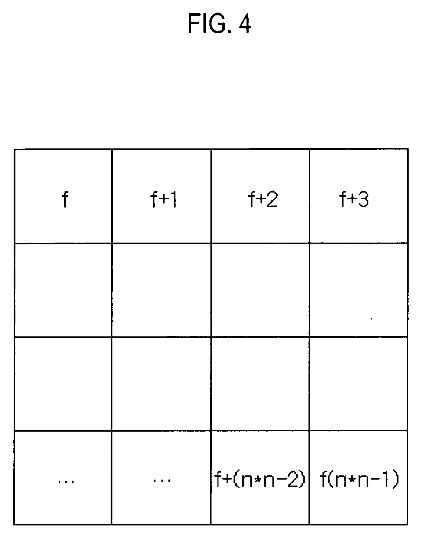

While the ultrasound images within an ECG cycle are displayed as shown in FIG. 4 or 5, the user can select one image (e.g., m^{th} ultrasound image) among the n x n ultrasound images through the user interface 90 such as a mouse, track ball and the like.

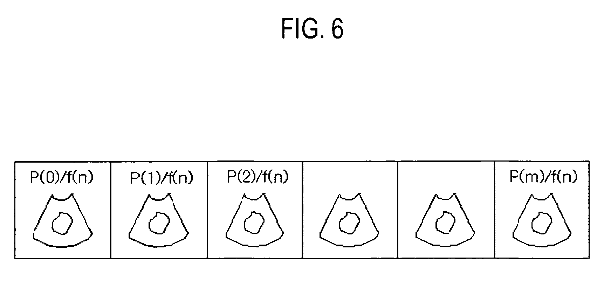

When the m^{th} ultrasound image is selected through the user interface 90, the m^{th} ultrasound images in the respective ECG cycles P(0), P(1), P(2) ... are selected from the second memory 60 and provided to the display unit 80. FIG. 6 shows an example of displaying the m^{th} ultrasound images in the respective ECG cycles. In such a case, the processor 50 compares the m^{th} ultrasound images in the neighboring ECG cycles. For example, the processor 50 may compare the m^{th} ultrasound image in the ECG cycle P(0) with the m^{th} ultrasound image in the ECG cycle P(1). Further, the processor 50 may compare the m^{th} ultrasound image in the ECG cycle P(1) with the m^{th} ultrasound image in the ECG cycle P(2). Then, a comparison result can be provided to the user through the display unit 80.

In accordance with the present invention, the user can obtain a more accurate diagnosis regarding the heart disease based on the ECG and ultrasound images provided from the ultrasound system.

An embodiment may be achieved in whole or in part by an ultrasound system, which includes: a ultrasound diagnostic unit for transmitting ultrasound signals to a target object in a periodic motion and forming ultrasound images based on receive signals reflected from the target object, wherein the ultrasound diagnostic unit is further configured to provide reception times of the receive signals corresponding to the ultrasound images; a first storage unit for storing the ultrasound images provided from the ultrasound diagnostic unit together with the reception times of the receive signals; a cycle setting unit for setting a cycle of motion of the target object; a processor for selecting the ultrasound images within a specific cycle among the ultrasound images stored in the first storage unit based on the reception times of the receive signals corresponding to the ultrasound images and start and end times of the specific cycle provided from the cycle setting unit, wherein the processor is configured to form an equal number of ultrasound images within each cycle based on the number of the selected ultrasound images; a second storage unit for storing the ultrasound images within each cycle provided from the processor in a regular order; and a display unit for simultaneously displaying the ultrasound images within each cycle.

Any reference in this specification to "one embodiment," "an embodiment," "example embodiment," etc. means that a particular feature, structure or characteristic described in connection with the embodiment is included in at least one embodiment of the invention. The appearances of such phrases in various places in the specification are not necessarily all referring to the same embodiment. Further, when a particular feature, structure or characteristic is described in connection with any embodiment, it falls within the purview of one skilled in the art to effectuate such feature, structure or characteristic in connection with other ones of the embodiments.

Although embodiments have been described with reference to a number of illustrative embodiments thereof, it should be understood that various other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to such variations and modifications in the component parts and/or arrangements, alternative uses will also be apparent to those skilled in the art.

## Claims

1. An ultrasound system, comprising:
an ultrasound diagnostic unit for transmitting ultrasound signals to a target object in a periodic motion and forming ultrasound images based on receive signals reflected from the target object, the ultrasound diagnostic unit further being configured to provide reception times of the receive signals corresponding to the ultrasound images;
a first storage unit for storing the ultrasound images provided from the ultrasound diagnostic unit together with the reception times of the receive signals;
a cycle setting unit for setting a cycle of motion of the target object;
a processor for selecting the ultrasound images within a specific cycle among the ultrasound images stored in the first storage unit based on the reception times of the receive signals corresponding to the ultrasound images and start and end times of the specific cycle provided from the cycle setting unit, the processor further being configured to form an equal number of ultrasound images within each cycle based on the number of the selected ultrasound images; and
a display unit for simultaneously displaying the ultrasound images within each cycle.

2. The ultrasound system of Claim 1, further comprising a second storage unit for storing the ultrasound images within each cycle provided from the processor in a regular order.

3. The ultrasound system of Claim 2, further comprising a user interface for selecting one ultrasound image among the ultrasound images displayed in the display unit by a user, wherein the processor extracts ultrasound images in respective cycles in the same order as an order of the selected ultrasound image from the second storage unit, and wherein the display unit simultaneously displays the extracted ultrasound images.

4. The ultrasound system of Claim 3, wherein the processor compares the ultrasound images in neighboring cycles among the extracted ultrasound images, and wherein the display unit displays the comparison result.

5. The ultrasound system of Claim 2, wherein the first and second storage units are configured with a single memory.

6. The ultrasound system of Claim 1, wherein the cycle setting unit includes:
an electrocardiogram (ECG) signal supply unit for providing ECG signals; and
an ECG cycle setting unit for analyzing the ECG signals provided from the ECG signal supply unit during a predetermined time period to set an ECG cycle, wherein the ECG cycle setting unit transmits start and end times of each ECG cycle to the processor.

7. The ultrasound system of Claim 6, wherein the processor sets the ultrasound images having reception times substantially equal to the start and end times of each ECG cycle as a start and an end image of each ECG cycle and counts the number of ultrasound images in each ECG cycle to display a predetermined number of ultrasound images in each ECG cycle, the processor being configured to remove extra ultrasound images except the predetermined number of ultrasound images when the number of ultrasound images within an ECG cycle is larger than the predetermined number, the processor further being configured to produce supplementary ultrasound images with interpolation based on neighboring ultrasound images to make the predetermined number of ultrasound images within an ECG cycle when the number of ultrasound images in an ECG cycle is smaller than the predetermined number.

8. A method of displaying ultrasound images, comprising:
a) transmitting ultrasound signals to a target object in a periodic motion and forming the ultrasound images based on receive signals reflected from the target object;
b) setting a cycle of motion of the target object;
c) forming a predetermined number of ultrasound images within a specific cycle based on reception times of the receive signals corresponding to the ultrasound images and start and end times of the specific cycle;
d) storing the ultrasound images within each cycle in a regular order; and
e) displaying the ultrasound images within each cycle simultaneously.

9. The method of Claim 8, further comprising:
selecting one ultrasound image among the ultrasound images displayed in the display unit by a user;
extracting the ultrasound images in respective cycles in the same order as an order of the selected ultrasound image from the second storage unit; and
displaying the extracted ultrasound images simultaneously.

10. The method of Claim 9, further comprising:
comparing ultrasound images in neighboring cycles among the extracted ultrasound images; and
displaying the comparison result.

11. The method of Claim 8, wherein the step b) includes:
providing ECG signals; and
analyzing the provided ECG signals during a predetermined time period to set an ECG cycle.

12. The method of Claim 8, wherein the step c) includes:
setting ultrasound images having reception times substantially equal to the start and end times of each ECG cycle as a start and an end image of each ECG cycle;
counting the number of ultrasound images in each ECG cycle to display a predetermined number of ultrasound images in each ECG cycle;
removing extra ultrasound images except the predetermined number of ultrasound images when the number of ultrasound images within an ECG cycle is larger than the predetermined number; and
producing supplementary ultrasound images with interpolation based on neighboring ultrasound images to make the predetermined number of ultrasound images within an ECG cycle when the number of ultrasound images in an ECG cycle is smaller than the predetermined number.
